## Europäisches Patentamt

## European Patent Office

## Office européen des brevets

(19)

(11) Numéro de publication: **0 079 305 B1**

## FASCICULE DE BREVET EUROPEEN

(12)

(45) Date de publication du fascicule du brevet:
**28.12.88**

(51) Int. Cl.⁴: **A 61 B 5/02**

(21) Numéro de dépôt: **82810446.3**

(22) Date de dépôt: **26.10.82**

---

(54) **Dispositif de mesure de la pression sanguine.**

---

(30) Priorité: **05.11.81 DE 3143871**

(43) Date de publication de la demande:
**18.05.83 Bulletin 83/20**

(45) Mention de la délivrance du brevet:
**28.12.88 Bulletin 88/52**

(84) Etats contractants désignés:
**CH DE FR GB LI**

(56) Documents cités:
**FR-A- 2 412 298**
**FR-A- 2 488 793**
**US-A- 2 827 040**
**US-A- 3 157 177**
**US-A- 3 508 537**
**US-A- 4 144 879**
**US-A- 4 312 359**

**PROCEEDINGS OF THE INSTRUMENT SOCIETY OF AMERICA, vol. 22, no. 4, 22nd Annual ISA Conference and Exhibit, Chicago, Ill. 11-14 septembre 1967, Pittsburgh, PA (US) ; M.T. LATEGOLA et al.:"Rapid and frequent measurement of human systolic and diastolic blood pressures", pages 1-8**

(73) Titulaire: **ASULAB S.A., Faubourg du Lac 6, CH-2501 Bienne (CH)**

(72) Inventeur: **Klaye, François, Port-Roulant 12A, CH-2000 Neuchatel (CH)**

(74) Mandataire: **de Montmollin, Henri et al, ICB Ingénieurs Conseils en Brevets SA Passage Max. Meuron 6, CH-2001 Neuchâtel (CH)**

---

## Description

La présente invention concerne un dispositif de mesure de la pression sanguine d'un sujet, comportant les divers éléments énumérés dans le préambule de la revendication 1.

Un dispositif de mesure de la pression sanguine connu par le brevet US 2,827,040 comporte un microphone pour détecter les bruits de Korotkoff produits par le sang circulant dans une artère. Le microphone est relié à un circuit de coïncidence par un formateur d'impulsions. Une manchette gonflable, destinée à être fixée à un bras d'un patient, est reliée à un réservoir d'air qui comporte une vanne d'échappement qui, lors des mesures, dirige un courant d'air sur un thermistor. Le thermistor sert à détecter les impulsions de pression dans le réservoir, et il est également relié au circuit de coïncidence par un formateur d'impulsions. Le dispositif comporte en outre un manomètre pour détecter la pression systolique et un manomètre pour détecter la pression diastolique. Chacun des deux manomètres est relié au réservoir d'air par une vanne. Le réservoir d'air est en outre relié par une vanne à un compresseur et, en plus, par une vanne de décharge à l'atmosphère ambiante. De plus, un dispositif de commande est prévu pour commander les différentes vannes.

Lors d'une mesure de la pression sanguine, la pression dans le réservoir d'air est tout d'abord augmentée. Pendant cette augmentation, des impulsions sont produites dans certains domaines de pression aussi bien par les bruits de Korotkoff que par les variations de pression et elles sont appliquées au circuit de coïncidence. Pendant les coïncidences d'impulsions produites en premier, c'est-à-dire en présence d'une pression basse, le manomètre destiné à la mesure de la pression diastolique est relié en permanence avec le réservoir d'air, de manière qu'il mesure et affiche la pression diastolique. Ensuite la pression est encore augmentée. Pendant les coïncidences d'impulsions se produisant à la pression la plus élevée, le manomètre servant à la mesure de la pression systolique est relié en permanence avec le réservoir d'air, ce qui mesure la pression systolique.

Ce dispositif connu présente l'inconvénient que l'appareil relié à la manchette doit comprendre quatre vannes ainsi que deux manomètres, et qu'il est donc de grandes dimensions et peu maniable. Un autre inconvénient résulte du fait que la détection des variations de pressions dues au pouls à l'aide d'un thermistor refroidi par un courant d'air est délicate et imprécise.

Un autre dispositif de mesure de la pression sanguine, connu par le brevet US 4,144,879, comporte une manchette gonflable destinée à être fixée au bras d'un patient. Il comporte en outre en capteur de pression et un circuit électronique produisant une double différenciation du signal électrique formé par le capteur de pression, de manière que la pression et les impulsions de pression produites par les pulsations inidividuelles du cœur puissent être détectées. Pour une mesure de la pression sanguine, la manchette est tout d'abord gonflée puis lentement dégonflée. Dans l'une des formes d'exécution décrites, un calculateur de processus et deux vannes à commande électrique sont prévues, dont l'une sert au dégonflage continu et l'autre au dégonflage par paliers. Le processus de dégonflage est commandé de manière que la pression commence par diminuer de manière continue. Ensuite, lorsque des impulsions de pression sont détectées, la pression est diminuée par paliers à chaque impulsion de pression. Lors du processus de dégonflage, les pressions systolique et diastolique sont déterminées lors de la détection d'impulsions de pression remplissant des critères prédéterminés.

Lors d'une mesure de la pression sanguine avec ce dispositif décrit par le brevet US 4,144,879 la pression dans la manchette est donc diminuée depuis la pression atteinte lors du gonflage jusqu'en dessous de la pression diastolique. Dans la phase dans laquelle la pression est diminuée par paliers, la diminution de pression est de environ 267 pascals (2 Torr) par pulsation cardiaque. La phase de dégonflage prend donc relativement beaucoup de temps. La réalisation de la mesure complète dure donc aussi relativement longtemps. Ceci représente un inconvénient, qui prend de l'importance surtout lorsque des mesures de pression sanguine doivent être effectuées souvent ou en série, comme c'est le cas par exemple lors des campagnes de don du sang. En outre la présence des deux vannes influence défavorablement le coût de l'appareil. Enfin, l'identification des pressions systolique et diastolique à l'aide d'impulsions de pression exclusivement est problématique et peut conduire facilement à des erreurs de mesure.

La demande de brevet FR-A-2488793 décrit un dispositif qui mesure successivement une valeur approximative et une valeur précise de la pression systolique, puis une valeur approximative et une valeur précise de la pression diastolique. Les valeurs approximatives sont mesurées pendant une diminution rapide la pression dans la manchette interrompue après chacune de ces mesures par une augmentation rapide de cette pression, suivie elle-même d'une diminution lente de la pression au cours de laquelle chaque valeur précise est mesurée.

La demande de brevet FR-A-2412298 décrit un dispositif qui, dans une première forme d'exécution, est semblable à celui qui vient d'être décrit. Dans une deuxième forme d'exécution, le dispositif fonctionne de manière similaire, mais les valeurs approximatives sont mesurées lors d'une augmentation rapide de la pression dans la manchette interrompue après chacune de ces mesures par une diminution rapide de cette pression, suivie elle-même par une augmentation lente de la pression au cours de laquelle chaque valeur précise est mesurée.

Les dispositifs décrits par ces deux dernières demandes de brevet ne sont pratiquement utilisables que s'ils comportent une pompe entraînée par un moteur pour assurer le gonflage de la manchette, ce moteur étant lui-même automatiquement commandé par le dispositif.

En effet, si un dispositif tel que celui décrit par la première des demandes ci-dessus était équipé d'une pompe à main, son utilisateur, qui est souvent le patient lui-même devrait surveiller très attentivement le dégonflage rapide pour l'interrompre immédiatement après chaque mesure approximative, et pour regonfler la manchette de la quantité voulue.

Dans le cas d'un dispositif semblable à la deuxième forme d'exécution de celui qui est décrit dans la deuxième des demandes ci-dessus, il n'est guère possible de gonfler rapidement ou lentement la manchette à l'aide d'une pompe à main.

Le but de la présente invention est de proposer un dispositif de mesure de la pression sanguine grâce auquel le temps nécesssaire pour une mesure est rendu aussi court que possible tout en permettant d'obtenir une grande précision de mesure, ce dispositif pouvant en outre être équipé indifféremment d'une pompe manuelle ou d'une pompe à moteur pour gonfler sa manchette.

Ce but est atteint par le dispositif de mesure de la pression sanguine revendiqué.

Pour être tout à fait clair, il faut noter que les expressions «pression sanguine» et «pression dans la chambre» doivent être comprises dans les revendications et dans la description comme étant des surpressions par rapport à la pression de l'air ambiant.

L'invention va maintenant être décrite à l'aide du dessin dans lequel:

- la figure 1 représente un schéma bloc d'un dispositif de mesure de la pression sanguine selon l'invention;

- la figure 2 est une partie d'un schéma bloc d'une variante du dispositif de la figure 1; et

- la figure 3 est un diagramme montrant la variation en fonction du temps de la pression sanguine et des signaux correspondant aux bruits de Korotkoff.

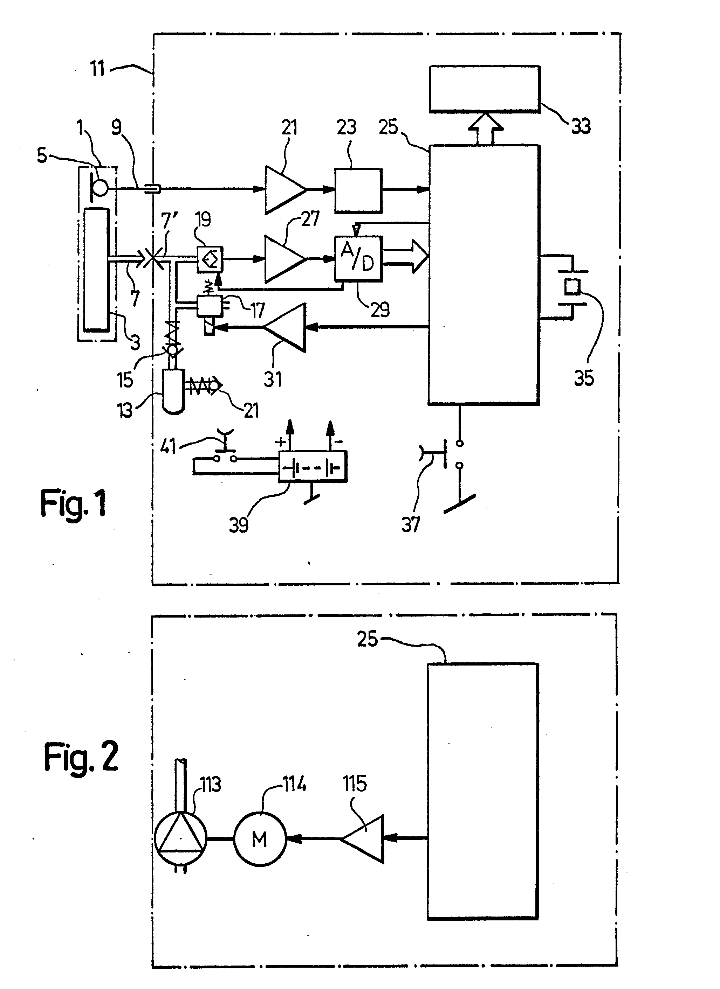

Le dispositif de mesure de la pression sanguine représenté à la figure 1 comporte une manchette 1 destinée à être fixée au bras d'un sujet et un appareil désigné comme un tout avec la référence 11. La manchette 1 comprend une chambre 3 en forme de poche en caoutchouc déformable et gonflable, ainsi qu'un microphone 5. Le microphone 5 est disposé de manière à capter les bruits produits par la circulation du sang dans le bras du sujet, notamment ceux qui sont connus sous le nom de bruits de Korotkoff et dont la détection permet la détermination des pressions diastolique et systolique du sujet.

La chambre 3 et le microphone 5 sont respectivement reliés à l'appareil 11 par une conduite flexible 7 et par un câble flexible 9 ainsi que, par exemple, par des connecteurs enfichables.

L'appareil 11 comporte un dispositif d'alimentation 39, qui comprend par exemple une batterie ou une alimentation par le réseau, et qui fournit les tensions nécessaires au fonctionnement des différents composants électroniques de l'appareil. Le dispositif 39 comporte un interrupteur 41 permettant d'enclencher et de déclencher l'alimentation. Une des bornes du dispositif 39 est en outre reliée à la masse de l'appareil 11.

L'appareil 11 comporte en outre une pompe 13 avec un corps de pompe, par exemple cylindrique, en matériau élastique. La chambre 3 est reliée à la pompe 13 par l'intermédiaire de la conduite 7, d'une conduite 7' montée dans l'appareil ainsi que d'une soupape antiretour 15. Une vanne de dégonflage à commande électrique 17 et un capteur de pression 19 sont également reliés à la conduite 7'. La pompe 13 comprend en outre une entrée d'air munie d'une soupape antiretour 21. Les deux soupapes antiretour 15 et 21 sont connectées de manière que, en pressant et en relachant alternativement et manuellement le corps de pompe, de l'air ambiant soit pompé dans la chambre 3.

Le microphone 5 est relié électriquement, par l'intermédiaire d'un amplificateur 21 comportant un filtre, à l'entrée d'un formateur d'impulsion 23 qui, lorsque les signaux qu'il reçoit dépassent un seuil déterminé, fournit des impulsions électriques d'amplitude et de forme constantes à un circuit de commande 25 relié à sa sortie. L'amplificateur 21 et le formateur d'impulsion 23 sont agencés de manière que ce dernier fournisse une impulsion pour chaque bruit de Korotkoff détecté par le microphone 5.

Le capteur de pression 19 comporte par exemple un circuit en pont formé d'éléments piézorésistifs. Il délivre en fonctionnement une tension proportionnelle à la pression dans la chambre 3, et il est relié par l'intermédiaire d'un amplificateur 27 à l'entrée d'un convertisseur analogique/digital 29. Ce dernier comporte une entrée de commande et huit sorties reliées au circuit de commande. En fonctionnement, le convertisseur analogique/digital 29 convertit le signal analogique qui lui est fourni par l'amplificateur 27 en un signal digital à huits bits et délivre ce signal digital en parallèle au circuit de commande 25 en réponse à un signal fourni à son entrée de commande par ce circuit de commande 25.

Le circuit de commande comporte, comme élément principal, un micro-ordinateur, qui peut être par exemple un micro-ordinateur du type 7502G de la maison Nippon Electric Company. Une sortie du circuit de commande 25 est reliée par l'intermédiaire d'un amplificateur 31 à la bobine actionnant de la vanne 17. Le circuit de commande 25, l'amplificateur 31 et la vanne sont agencés de manière que cette dernière, en fonctionnement, est toujours entièrement fermée ou entièrement ouverte.

D'autres sorties du circuit de commande 25 sont reliées à un dispositif d'affichage 33, qui comporte des éléments à cristal liquide ou des éléments semblables pour l'affichage sous forme numérique des pressions et de la fréquence du pouls, ainsi que pour l'affichage de symboles représentants des états de fonctionnement déterminés. Le circuit de commande 25 est en outre relié à un résonateur à quartz 35, qui sert de générateur de cadence et dont la fréquence de résonance est, par exemple, de 32 KHz. Une borne du circuit de commande 25 est reliée à la masse de l'appareil 11 par l'intermédiaire d'un bouton poussoir de remise à zéro 37.

La figure 2 représente une partie d'une variante de ce dispositif de mesure de la pression sanguine dans laquelle la pompe 13 actionnée manuellement est remplacée par une pompe 113 entraînée par un moteur électrique 114. Des soupapes correspondant aux soupapes antiretour 13 et 21 pourraient éventuellement être prévues. Cependant il est possible de renoncer à ces soupapes si la pompe 113 est agencée de manière que la liaison entre son entrée et sa sortie d'air soit interrompue de manière étanche en position de repos. Pour le reste, le dispositif de mesure de la pression sanguine comprend les mêmes éléments que ceux qui sont représentés à la figure 1.

Si une telle pompe 113 et un tel moteur 114 sont prévus, leur commande peut être effectuée par un simple interrupteur actionné manuellement. Cependant, de préférence, le circuit de commande 25 peut être programmé pour fournir des signaux qui enclenchent et déclenchent le moteur 114 par l'intermédiaire d'un amplificateur 115.

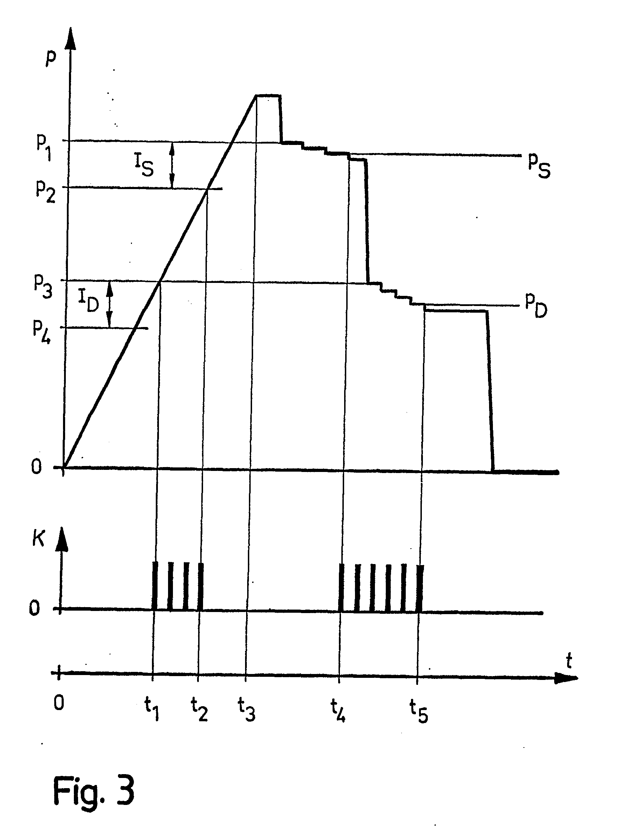

Le déroulement de la mesure va maintenant être décrit à l'aide de la figure 3 dans un cas où le dispositif est équipé de la pompe 113 et du moteur 114 décrits par la figure 2. Il sera rendu évident par cette description que la mesure proprement dite se déroule exactement de la même manière lorsque le dispositif est semblable à celui de la figure 1, c'est-à-dire lorsqu'il comporte la pompe 13 à actionnement manuel.

La partie supérieure de la figure 3 représente le diagramme de la variation en fonction du temps de la pression p régnant dans la chambre 3 et mesurée par le capteur de pression 19. La partie inférieure de cette figure 3 représente par un trait vertical chaque impulsion fournie par le formateur d'impulsions 23 au circuit de commande 25 en réponse à un bruit de Korotkoff.

Pour exécuter une mesure, la manchette 1 est fixée à un membre du sujet, généralement à un de ses bras. En outre, la chambre 3 et le microphone 5 sont reliés à l'appareil 11 si cette liaison n'est pas déjà faite.

Après la fixation de la manchette 1, l'appareil est mis en marche en fermant l'interrupteur 41. Au temps t = 0, le circuit de commande 25 enclenche le moteur 114, et la pompe 113 commence à envoyer de l'air dans la chambre 3, de manière que la pression dans cette chambre 3 augmente. La vanne 17 est fermée par le circuit de commande 25 à l'enclenchement de l'appareil ou au début du pompage. Pendant ce pompage, la pression p dans la chambre 3 est affichée en permanence par le dispositif d'affichage 33. Cette pression p augmente régulièrement, à une vitesse qui est avantageusement au moins approximativement constante et qui est par exemple de 2 à 5 KPa par seconde.

Le premier signal de bruit de Korotkoff est fourni au circuit de commande 25 par le microphone 5, l'amplificateur 21 et le formateur 23 à l'instant $t_1$. La valeur $p_3$ de la pression mesurée à cet instant $t_1$ est supérieure ou égale à la valeur de la pression diastolique du sujet. Cette pression $p_3$ est la pression de la limite supérieure d'un domaine de pression dans lequel se trouve la pression diastolique $p_D$ du sujet et qui sera appelé domaine de pression diastolique $I_D$.

La pression p continue à augmenter, et, pendant un certain temps, le circuit de commande 25 continue à recevoir des signaux de bruit de Korotkoff.

A l'instant $t_3$, le circuit de commande 25 constate que aucun bruit de Korotkoff n'a été émis depuis un certain temps, égal à trois secondes par exemple, ou qu'une pression maximum prédéterminée a été atteinte. La pression $p_2$ mesurée dans la chambre 3 à l'instant $t_2$ où le dernier bruit de Korotkoff a été produit est inférieure ou égale à la valeur de la pression systolique du sujet. Cette pression $p_2$ est la pression de la limite inférieure d'un domaine de pression dans lequel se trouve la pression systolique $p_S$ du sujet et qui sera appelé domaine de pression systolique $I_S$.

La valeur des limites inférieure, respectivement supérieure des domaines de pression $I_D$ et $I_S$ peut être déterminée en mesurant la pression dans la chambre 3 à des instants séparés par des intervalles de temps courts, de 0,1 seconde par exemple, et en mémorisant toutes les valeurs mesurées. Le circuit de commande 25 peut ainsi sélectionner parmi toutes ces valeurs mémorisées celle qui a été mesurée 1,5 seconde, par exemple, avant l'instant $t_1$, et prendre cette valeur sélectionnée comme valeur $p_4$ de la limite inférieure du domaine de pression diastolique $I_D$. De même, le circuit de commande peut sélectionner la valeur mémorisée 1,5 seconde après l'instant $t_2$ comme valeur $p_1$ de la limite supérieure du domaine de pression systolique $I_S$.

La largeur des domaines de pression $I_D$ et $I_S$ peut également être fixe et prédéterminée, et être mémorisée dans le circuit de commande. La valeur $p_4$ de la limite inférieure du domaine $I_D$ est alors obtenue en soustrayant cette valeur mémorisée de la valeur $p_3$ mesurée à l'instant $t_1$, et la valeur $p_1$ de la limite supérieure du domaine $I_S$ est obtenue en ajoutant cette valeur mémorisée à la valeur $p_2$ mesurée à l'instant $t_2$.

A l'instant $t_3$, le circuit de commande 25 déclenche le moteur 114 entraînant la pompe 113. Après cet instant $t_3$, le circuit de commande 25 attend pendant un temps prédéterminé de 1 à 2 secondes que la pression se stabilise, et déclenche un processus de dégonflage pendant lequel la vanne 17 est alternativement complètement ouverte et complètement fermée par le circuit de commande 25, de manière que la pression dans la chambre 3 diminue par sauts.

Au début de ce processus de dégonflage, le circuit de commande 25 ouvre la vanne 17 de manière que la pression dans la chambre 3 diminue par un saut unique jusqu'à la valeur $p_1$ environ. Ensuite, le circuit de commande ouvre et ferme la vanne 17 de manière que cette pression diminue par sauts ayant une amplitude d constante et prédéterminée. Cette amplitude d peut être choisie dans un domaine allant par exemple de 200 pascals (1,5 Torr) jusqu'à 600 pascals (4,5 Torr). Cette amplitude d est par exemple de 400 pascals (3 Torr). Le circuit de commande 25 commande la vanne 17 de manière que les sauts de dégonflage se suivent à des intervalles de temps prédéterminés, qui peuvent avoir une durée de 1 à 2 secondes, par exemple 1,5 seconde.

Après un certain nombre de diminutions de la pression de la quantité d, deux dans l'exemple de la figure 3, le premier bruit de Korotkoff est détecté, à l'instant $t_4$ qui coïncide avec une nouvelle diminution de pression. A partir de cet instant $t_4$, les baisses de pressions sont déclenchées par les bruits de Korotkoff, et non plus à invervalles de temps prédéterminés. En outre, lors du bruit de Korotkoff suivant celui qui a été détecté à l'instant $t_4$, le circuit de commande 25 ouvre la vanne 17 de manière que la pression diminue par un saut unique jusqu'à la valeur $p_3$ environ. Ensuite, la pression est à nouveau diminuée par sauts d'amplitude d en synchronisme avec les bruits de Korotkoff.

Le dernier bruit de Korotkoff arrive à l'instant $t_5$. Le circuit de commande 25 maintient la vanne 17 fermée après la baisse de pression produite par ce dernier bruit de Korotkoff pendant une durée prédéterminée de 3 à 6 secondes, et ouvre ensuite la vanne 17 de manière que la chambre 3 se dégonfle complètement.

La pression qui régnait dans la chambre 3 avant le saut de pression qui précède l'arrivée du premier bruit de Korotkoff à l'instant $t_4$ est mémorisée dans une mémoire du circuit de commande 25 comme pression systolique $p_S$. De même, la pression qui régnait dans la chambre 3 avant le saut de pression produit par le dernier bruit de Korotkoff est mémorisée comme pression diastolique $p_D$. Ensuite, la pression systolique et la pression diastolique sont affichées simultanément dans des zones différentes de l'organe d'affichage 33, sous forme numérique.

Le circuit 25 détermine en outre la fréquence du pouls, la mémorise et l'affiche dans une zone particulière de l'organe d'affichage 33. Lorsque la personne qui utilise l'appareil a lu les valeurs mesurées, l'appareil peut être dèclenché par l'ouverture de l'interrupteur 41. La vanne 17 reste ouverte après le déclenchement de l'appareil.

Lorsque l'interrupteur 41 est à nouveau fermé, pour remettre en service l'appareil pour effectuer une autre mesure, le déroulement du programme de mesure qui a été décrit ci-dessus est à nouveau enclenché.

Si une mesure doit être interrompue et recommencée pour une raison quelconque, une courte pression sur le commutateur de remise à zéro 37 provoque le recommencement du programme de mesure depuis son début.

Pendant le déroulement de la mesure, le circuit de commande 25 exécute encore certains contrôles et opérations destinés à éviter des erreurs de mesure. Par exemple, pendant le processus de pompage, il empêche que les bruits de Korotkoff provoquent une ouverture de la vanne 17. Si le premier signal de bruit de Korotkoff apparaît déjà peu de temps après la fin du processus de pompage, par exemple avant l'écoulement d'un intervalle de temps de 1 à 4 secondes à partir de la fin du processus de pompage, le processus de dégonflage n'est pas enclenché, ou bien il est à nouveau interrompu et le dispositif d'affichage 33 signale que la chambre 3 doit être encore gonflée. En outre les signaux produits par le microphone 5 ne sont reconnus comme de vrais bruits de Korotkoff que s'ils sont séparés par des intervalles de temps ayant une valeur inférieure à une valeur prédéterminée de l'ordre de 1 à 2,5 secondes.

Dans la phase de gonflage de la chambre 3, la pression est augmentée avec une vitesse relativement grande. Les deux domaines de pression $I_S$ et $I_D$ dans lesquels les pressions systolique et diastolique se trouvent sont donc déterminés rapidement. Dans la phase de dégonflage, seules une partie du domaine de pression systolique $I_S$ et une partie du domaine de pression diastolique $I_D$ sont parcourues avec la vitesse moyenne relativement faible qui est nécessaire à l'obtention d'une valeur précise des pressions systolique $p_S$ et diastolique $p_D$. Il en résulte que le temps nécessaire à l'exécution de la mesure est sensiblement plus court que celui qui est nécessaire pour effectuer une mesure avec un dispositif connu.

Le fait que les baisses de pressions sont synchronisées avec l'arrivée des bruits de Korotkoff permet en outre de garantir que au moins la pression diastolique est toujours mesurée avec la même résolution et indépendamment de la fréquence du pouls.

Bien qu'il soit avantageux de procéder au dégonflage de la chambre 3 par sauts, comme cela a été décrit ci-dessus, il serait sans autre possible de dégonfler cette chambre 3 de manière que la pression soit réduite continuellement et lentement à partir de la valeur limite supérieure du domaine de pression systolique $I_S$ pour parcourir au moins une partie de ce domaine de pression systolique, jusqu'à l'obtention de la pression systolique, et que, après l'obtention de la pression systolique $p_S$, la pression soit réduite par un saut unique jusqu'à la valeur limite supérieure du domaine de pression diastolique $I_D$, et que ce dernier domaine soit ensuite parcouru lentement et de manière continue jusqu'à ce que la pression diastolique $p_D$ soit déterminée.

Dans les parties de la phase de dégonflage pendant lesquelles la pression est diminuée lentement pour déterminer la pression systolique, respectivement diastolique, la vitesse de variation de la pression est sensiblement plus petite que pendant la phase de gonflage pendant lesquelles les domaines de pression $I_S$ et $I_D$ sont déterminés.

Il faut encore noter qu'il est possible d'utiliser un récipient contenant de l'air ou un autre gaz sous pression et relié avec la chambre gonflable de la manchette par une vanne à commande électrique comme soure de pression à la place d'une pompe.

Dans toutes les variantes décrites ci-dessus, la vitesse moyenne de variations de la pression p est sensiblement plus élevée pendant la détermination des domaines de pressions systolique $I_S$ et diastolique $I_D$ que pendant la détermination des pressions systolique $p_S$ et diastolique $p_D$ elles-mêmes, indépendamment du fait que la pression varie par paliers ou de manière continue.

## Revendications

1. Dispositif de mesure de la pression sanguine d'un sujet, comportant une manchette (1) comprenant une chambre déformable (3) et un microphone (5) et destinée à être fixée autour d'un membre du sujet, le dispositif comportant en outre des premiers moyens (13, 15, 17; 113, 15, 17) pour modifier la pression dans la chambre (3) dans une plage comportant au moins la pression systolique ($p_S$) et la pression diastolique ($p_D$) du sujet, lesdits premiers moyens (13, 15, 17; 113, 15, 17) comportant des deuxièmes moyens (13, 15; 113, 15) pour augmenter la pression dans la chambre (3) et des troisièmes moyens (17) pour diminuer la pression dans la chambre (3), des quatrièmes moyens (19) pour produire un signal de pression en réponse à la pression dans la chambre (3), et des cinquièmes moyens (25) pour commander au moins lesdits troisièmes moyens (17) et pour déterminer les pressions systolique ($p_S$) et diastolique ($p_D$) du sujet en réponse audit signal de pression et aux bruits de Korotkoff captés par ledit microphone (5), lesdits cinquièmes moyens (25) étant agencés de manière à déterminer chacune desdites pressions systolique ($p_S$) et diastolique ($p_D$) en deux étapes distinctes, les premières lesdites étapes comportant respectivement la détermination d'un domaine de pression systolique ($I_S$) comprenant ladite pression systolique ($p_S$) et la détermination d'un domaine de pression diastolique ($I_D$) comprenant ladite pression diastolique ($p_D$), et les deuxièmes desdites étapes comportant respectivement une variation de la pression dans la chambre (3) entre les limites dudit domaine de pression systolique ($I_S$) et une variation de la pression dans la chambre (3) entre les limites dudit domaine de pression diastolique, ainsi que la détermination précise de ladite pression systolique ($p_S$) pendant ladite variation de la pression dans la chambre (3) entre les limites dudit domaine de pression systolique ($I_S$) et la détermination précise de ladite pression diastolique ($p_D$) pendant ladite variation de la pression dans la chambre (3) entre les limites dudit domaine de pression diastolique ($I_D$), caractérisé par le fait que lesdits cinquièmes moyens (25) sont en outre agencés de manière que lesdites premières étapes soient réalisées à la suite l'une de l'autre pendant une augmentation de la pression dans la chambre (3) entre les limites de ladite plage de pression, de manière que lesdits troisièmes moyens (17) soient commandés après la fin de ladite augmentation de pression pour provoquer une diminution de pression dans la chambre (3), et de manière que lesdites deuxièmes étapes soient réalisées à la suite l'une de l'autre pendant ladite diminution de pression dans la chambre (3).

2. Dispositif selon la revendication 1, caractérisé par le fait que lesdits cinquièmes moyens (25) sont en outre agencés de manière que la pression dans la chambre (3) soit rapidement diminuée jusqu'à la valeur maximum (p1) du domaine de pression systolique ($I_S$) à la fin de ladite augmentation de la pression dans la chambre (3), que la pression dans la chambre (3) soit rapidement diminuée jusqu'à la valeur maximum (p3) du domaine de pression diastolique ($I_D$) après que la pression systolique ($p_S$) du sujet a été déterminée lors de la deuxième étape de sa détermination, et que la pression dans la chambre (3) soit rapidement diminuée jusqu'à la valeur inférieure de ladite plage de pression après que la pression diastolique ($p_D$) du sujet a été déterminée lors de la deuxième étape de sa détermination.

3. Dispositif selon la revendication 1 ou 2, caractérisé par le fait que lesdits cinquièmes moyens sont en outre agencés de manière que la pression dans la chambre (3) décroisse par sauts d'amplitude prédéterminée pendant ladite diminution de la pression dans la chambre (3), lesdits sauts étant effectués à intervalles de temps prédéterminés avant l'apparition du premier son de Korotkoff, et en réponse auxdits sons de Korotkoff après l'apparition dudit premier son de Korotkoff.

4. Dispositif selon l'une des revendications 1 à 3, caractérisé par le fait que lesdits deuxièmes moyens (13, 15; 113, 15) comportent une pompe (13) actionnable manuellement pour augmenter la pression dans la chambre (3).

5. Dispositif selon l'une des revendications 1 à 3, caractérisé par le fait que lesdits deuxièmes moyens (13, 15; 113, 15) comportent une pompe (113) entraînée par un moteur (114), et que lesdits cinquièmes moyens (25) sont en outre agencés de manière à commander ledit moteur (114) pour provoquer ladite augmentation de la pression dans la chambre (3).

## Claims

1. A device for measuring the blood pressure of a subject, which comprises a cuff (1) having a deformable chamber (3) and a microphone (5) and intended to be fitted round a member of the subject, the device further comprising first means (13, 15, 17; 113, 15, 17) for modifying the pressure in the chamber (3) within a gamut that includes at least the subject's systolic pressure ($p_S$) and diastolic pressure ($p_D$), said first means (13, 15, 17; 113, 15, 17) including second means (13, 15; 113, 15) for increasing the pressure in the chamber (3) and third means (17) for reducing the pressure in the chamber (3), fourth means (19) for producing a pressure signal in response to the pressure in the chamber (3), and fifth means (25) for controlling at least said third means (17) and for determining the subject's systolic and diastolic pressure ($p_S$, $p_D$) in response to said pressure signal and to the Korotkoff sounds picked up by said microphone (5), said fifth means (25) being adapted to determine each of said systolic and diastolic pressure ($p_S$, $p_D$) in

two separate stages, the first stages respectively involving determining a systolic pressure range ($I_S$) that encompasses said systolic pressure ($p_S$) and determining a diastolic pressure range ($I_D$) that encompasses said diastolic pressure ($p_D$), and the second stages respectively involving varying the pressure in the chamber (3) within the limits of said systolic pressure range ($I_S$) and varying the pressure in the chamber (3) within the limits of said diastolic pressure range, as well as accurately determining said systolic pressure ($p_S$) while the pressure is being varied in the chamber (3) within the limits of said systolic pressure range ($I_S$) and accurately determining said diastolic pressure ($p_D$) while the pressure in the chamber (3) is being varied within the limits of said diastolic pressure range ($I_D$), characterized in that said fifth means (25) are also so arranged that said first stages are carried out one after the other during a rise in the pressure in the chamber (3) within the limits of said pressure gamut, that said third means (17) are controlled after said pressure rise has ended to cause a pressure drop in the chamber (3), and that said second stages are carried out one after the other during said pressure drop in the chamber (3).

2. A device according to claim 1, characterized in that said fifth means (25) are also so arranged that the pressure in the chamber (3) rapidly drops to the maximum value (p1) of the systolic pressure rang ($I_S$) at the end of said rise in pressure in the chamber (3), that the pressure in chamber (3) is rapidly decreased to the maximum value (p3) of the diastolic pressure range ($I_D$) after the subject's systolic pressure ($p_S$) has been determined during the second stage of its determination, and that the pressure in the chamber (3) is rapidly decreased to the lower valuer of said pressure gamut after the subject's diastolic pressure ($p_D$) has been determined during the second stage of its determination.

3. A device according to claim 1 or 2, characterized in that said fifth means are also so arranged that the pressure in the chamber (3) decreases in jumps of predetermined amplitude during said pressure drop in the chamber (3), said jumps being carried out at predetermined time intervals before the appearance of the first Korotkoff sound, and in response to said Korotkoff sounds after the appearance of said first Korotkoff sound.

4. A device according to anyone of claims 1 and 3, characterized in that said second means (13, 15; 113, 15) include a mnually operable pump (13) for increasing the pressure in the chamber (3).

5. A device according to anyone of claims 1 to 3, characterized in that said second means (13, 15; 113, 15) include a pump (113) driven by a motor (114), and in that said fifth means (25) are also arranged to control said motor (114) to cause said pressure rise in the chamber (3).

**Patentansprüche**

1. Vorrichtung zur Messung des Blutdrucks einer Person, bestehend aus einer Manschette (1), die eine verformbare Kammer (3) und ein Mikrophon (5) umfasst und die bestimmt ist, um um ein Glied der Person befestigt zu werden, wobei die Vorrichtung ferner erste Mittel (13, 15, 17; 113, 15, 17) umfasst, um den Druck in Kammer (3) innerhalb eines Bereichs zu verändern, der mindestens den systolischen Druck ($p_S$) und den diastolischen Druck ($p_D$) der Person umfasst, wobei die genannten ersten Mittel (13, 15, 17; 113, 15, 17) zweite Mittel (13, 15; 113, 15) umfassen, um den Druck in Kammer (3) zu erhöhen und dritte Mittel (17), um den Druck in Kammer (3) zu vermindern, vierte Mittel (19), um ein Drucksignal im Ansprechen auf den Druck in Kammer (3) zu erzeugen, und fünfte Mittel (25), um mindestens die genannten dritten Mittel (17) zu steuern und um des systolischen Druck ($p_S$) und den diastolischen Druck ($p_D$) der Person im Ansprechen auf den genannten Drucksignal und auf die vom genannten Mikrophon (5) erfassten Korotkoff-Geräusche zu bestimmen, wobei die genannten Fünften Mittel (25) so ausgestattet sind, dass sie den genannten systolischen Druck ($p_S$) und diastolischen Druck ($p_D$) jeweils in zwei verschiedenen Etappen bestimmen, wobei die ersten der genannten Etappen respektiv das Bestimmen eines den genannten systolischen Druck ($p_S$) umfassenden Druckbereichs ($I_S$) und das Bestimmen eines den genannten diastolischen Druck ($p_D$) umfassenden Druckbereichs ($I_D$) umfassen, und die zweiten genannten Etappen respektiv eine Druckänderung in Kammer (3) in den Grenzen des genannten systolischen Druckbereichs ($I_S$) und eine Druckänderung in Kammer (3) in den Grenzen des genannten diastolischen Druckbereichs ($I_D$) umfassen, sowie das genaue Bestimmen des genannten systolischen Drucks ($p_S$) während der genannten Druckänderung in Kammer (3) in den Grenzen des genannten systolischen Druckbereichs ($I_S$) und das genaue Bestimmen des genannten diastolischen Drucks ($p_D$) in den Grenzen des genannten diastolischen Druckbereichs ($I_D$), dadurch gekennzeichnet, dass die genannten fünften Mittel (25) ferner ao ausgestattet sind, dass die genannten ersten Etappen nacheinander während einer Erhöhung des Drucks in Kammer (3) innerhalb des genannten Druckbereichs ausgeführt werden, so dass die genannten dritten Mittel (17) nach der Ende der genannten Druckerhöhung angesteuert werden, um eine Druckverminderung in Kammer (3) zu bewirken, und so dass die genannten zweiten Etappen nacheinander während der genannten Druckverminderung in Kammer (3) ausgeführt werden.

2. Vorrichtung gemäss Anspruch 1, dadurch gekennzeichnet, dass die genannten fünften Mittel (25) ferner so ausgestattet sind, dass der Druck in Kammer (3) am Ende der genannten Druckerhöhung schnell bis auf den Maximalwert (p1) des systolischen Druckbereichs ($I_S$) vermindert wird, dass der Druck in Kammer (3) schnell bis auf den Maximalwert (p3) des diastolischen Druckbereichs ($I_D$) vermindert wird, nachdem der systolische Druck ($p_S$) der Person im Verlauf der zweiten Etappe seiner Bestimmung erfasst worden ist, und dass der Druck in Kammer (3) schnell bis auf den unteren Grenzwert des genannten Druckbereichs vermindert wird, nachdem der diastolische Druck ($p_D$) der Person im Verlauf der zweiten Etappe seiner Bestimmung erfasst worden ist.

3. Vorrichtung gemäss Anspruch 1 oder 2, dadurch gekennzeichnet, dass die genannten fünften Mittel ferner so ausgestattet sind, dass der Druck in Kammer (3) während der genannten Druckverminderung durch Sprünge vorbestimmter Grösse abnimmt, wobei die genannten Sprünge in vorbestimmten Zeitabständen vor dem Erscheinen der ersten Korotkoff-Tons und jeweils im Ansprechen auf die Korotkoff-Töne nach dem Erscheinen des ersten Korotkoff-Tons ausgeführt werden.

4. Vorrichtung gemäss einem der Ansprüche 1 bis 3, dadurch gekennzeichnet, dass die genannten zweiten Mittel (13, 15; 113, 15) eine von Hand betätigbare Pumpe (13) umfassen, um den Druck in Kammer (3) zu erhöhen.

5. Vorrichtung gemäss der Ansprüche 1 bis 3, dadurch gekennzeichnet, dass die genannten zweiten Mittel (13, 15; 113, 15) eine mittels Motor (114) angetriebene Pumpe (113) umfassen, und dass die genannten fünften Mittel (25) ferner so ausgestattet sind, dass der genannte Motor (114) angesteuert wird, umd die genannte Druckerhöhung in Kammer (3) zu bewirken.

Fig. 1

Fig. 2

Fig. 3